Europäisches Patentamt

European Patent Office

Office européen des brevets

(19)

(11) Veröffentlichungsnummer: **0 229 340 B1**

## (12) EUROPÄISCHE PATENTSCHRIFT

(45) Veröffentlichungstag der Patentschrift: **06.03.91**

(51) Int. Cl.5: **C07C 291/04**

(21) Anmeldenummer: **86117376.3**

(22) Anmeldetag: **13.12.86**

Die Akte enthält technische Angaben, die nach dem Eingang der Anmeldung eingereicht wurden und die nicht in dieser Patentschrift enthalten sind.

(54) Verfahren zur Herstellung von flüssigen, hochkonzentrierten Aminoxiden.

(30) Priorität: **19.12.85 DE 3545152**

(43) Veröffentlichungstag der Anmeldung:
**22.07.87 Patentblatt 87/30**

(45) Bekanntmachung des Hinweises auf die Patenterteilung:
**06.03.91 Patentblatt 91/10**

(84) Benannte Vertragsstaaten:
**DE ES FR GB IT**

(56) Entgegenhaltungen:

| | |
|---|---|
| DE-A- 2 557 456 | DE-A- 3 014 510 |
| DE-A- 3 145 735 | DE-B- 1 221 645 |
| DE-B- 1 232 590 | DE-B- 1 236 517 |
| DE-B- 1 518 104 | GB-A- 2 032 422 |
| US-A- 3 366 632 | US-A- 4 275 236 |
| US-A- 4 411 893 | |

(73) Patentinhaber: **HOECHST AKTIENGESELL-SCHAFT**
**Postfach 80 03 20**
**W-6230 Frankfurt am Main 80(DE)**

(72) Erfinder: **Blaschke, Günter, Dr.**
**Bajuwarenstrasse 36**
**W-8261 Winhöring(DE)**
Erfinder: **Kleber, Rolf, Dr.**
**Am Trieb 41**
**W-6078 Neu-Isenburg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von flüssigen, hochkonzentrierten Aminoxiden durch Oxidation der entsprechenden tertiären Amine in wäßriger Phase mit Wasserstoffperoxid ohne Zusatz organischer Lösungsmittel und ohne das Auftreten einer Gelphase.

Aminoxide sind unter anderem bekannte Zusätze zu Faserpräparationsmitteln. Die Herstellung von Aminoxiden durch Oxidation mit Wasserstoffperoxid ist dem Fachmann wohlbekannt. Die Umsetzung von tertiären Aminen der Formel $R^1R^2R^3N$, worin $R^1$ und $R^2$ kurzkettige Alkylreste (Methyl-und Ethylreste) und $R^3$ einen langkettigen Alkylrest mit 10 bis 20 c-Atomen darstellen, wird beschrieben in den US-Patentschriften 3 215 741 und 3 283 007. Dort sind auch die Schwierigkeiten aufgezeigt, die sich dabei ergeben (Spalte 1, Zeile 39 ff. der erstgenannten und Spalte 1, Zeile 22 ff. der letztgenannten Druckschrift): Werden konzentrierte Wasserstoffperoxid-Lösungen eingesetzt, so erstarrt die Reaktionsmischung zu einem Gel, das eine ausreichende Durchmischung und damit eine vollständige Umsetzung unmöglich macht. Verwendet man aber verdünnte Wasserstoffperoxid-Lösungen, so wird es schwierig, eine vollständige Umsetzung des Amins zu erzielen. Nicht-umgesetztes Amin ist aber im Produktgemisch höchst unerwünscht. Auch wird die Reaktionsgeschwindigkeit erheblich herabgesetzt und ferner werden die Aminoxide als stark verdünnte Lösungen erhalten. Zur Abhilfe wird vorgeschlagen, mit relativ konzentriertem Wasserstoffperoxid zu arbeiten und das sich bildende Gel durch Zusatz gewisser Wassermengen während der Reaktion zu zerstören. Auf diese Weise kann man jedoch höchstens 40 gew.-%ige Aminoxide dieses Typs in Wasser erhalten.

Um diese Schwierigkeit zu umgehen, wird in der deutschen Offenlegungsschrift 2 557 456 eine Arbeitsweise beschrieben, bei der eine genau begrenzte Wassermenge dem Reaktionsgemisch zusammen mit konzentriertem Wasserstoffperoxid zugesetzt wird, die so bemessen ist, daß der Anteil im Produktgemisch 20 bis 30 Gew.-% beträgt, da nach den Angaben dieser Beschreibung im genannten Konzentrationsbereich für Aminoxide, die zwei kurzkettige und einen langkettigen Alkylrest aufweisen, ein Viskositätsminimum durchlaufen wird. Bei Nacharbeitungen zeigt sich jedoch, daß auch in diesem Konzentrationsbereich eine Gelbildung gegeben ist und diese nur verhindert werden kann, wenn man hohe Scherkräfte einwirken läßt. Auch ist die genaue Einhaltung der erforderlichen Wasserkonzentration ein Hindernis für eine einfache Herstellungsmethode.

Nach einem aus US-A-4 275 236 bekannten Verfahren, können Aminoxide aus tertiären Aminen, die zwei Substituenten mit je mindestens 4 C-Atomen aufweisen, von denen jeder Substituent eine freie OH-Gruppe am ß-Kohlenstoffatom enthält, ohne Gelbildung in konzentrierter wäßriger Lösung erhalten werden. Das ist jedoch nicht möglich, wenn zwei kurzkettige Reste mit je bis zu 3 C-Atomen am Stickstoffatom des tertiären Amins gebunden sind, auch wenn diese Reste je eine freie OH-Gruppe aufweisen.

Es besteht ein Bedürfnis nach einem Verfahren, das hochkonzentrierte Aminoxide in flüssiger und gießfähiger Form liefert, bei dem andere tertiäre Amine als nach US-A-4 275 236 eingesetzt werden können, insbesondere solche, die keine Substituenten mit freien OH-Gruppen oder nur einen kurzkettigen Substituent mit einer freien OH-Gruppe aufweisen.

Diesem Bedürfnis wird die vorliegende Erfindung gerecht durch ein Verfahren der eingangs genannten Art, das dadurch gekennzeichnet ist, daß ein tertiäres Amin der Formel $R^1R^2R^3N$, worin $R^1$ und $R^2$, gleich oder verschieden, Alkyl- oder Alkenylreste mit 8 bis 18 C-Atomen oder Reste der Formel $R^1(OCH_2CH_2)n'$ in der $R^1$ die vorstehend angegebene Bedeutung hat und $n = 1$ bis 5 ist, bedeuten sowie $R^3 = CH_3$, $C_2H_5$, $C_2H_4OH$ oder $C_3H_6OH$ ist, oxidiert wird und die Konzentration des Wasserstoffperoxids so gewählt wird, daß das Aminoxid-Endprodukt der Formel $R^1R^2R^3N \rightarrow O$ in einer Konzentration von 40 bis 98 Gew.-% im Wasser vorliegt.

Der erfindungsgemäßen Verfahrensweise liegt die überraschende Erkenntnis zugrunde, daß bei der Umsetzung von tertiären Aminen, die zwei langkettige C-Ketten-Reste ohne freie OH-Gruppen und einen kurzkettigen C-Ketten-Rest aufweisen, mit Wasserstoffperoxid in wäßriger Phase über den gesamten Konzentrationsbereich eine Gelphase nicht beobachtet wird. Dies war überraschend, da die Fachwelt bis in die jüngste Zeit nicht annehmen konnte, daß die Herstellung von Aminoxiden dieses Typs auf dem eingeschlagenen Weg möglich sei. So ist noch vor kurzem angegeben worden, Diheptylmethylaminoxid mit wäßrigem Wasserstoffperoxid in Tetrahydrofuran zu oxidieren und das Lösungsmittel dann zu entfernen, um eine konzentrierte Einstellung zu erhalten (US-Patentschrift 4 411 893, Beispiel 7). Eine solche Entfernung eines organischen Lösungsmittels im technischen Maßstab ist jedoch bei der starken Schaumneigung solcher Aminoxide ein nahezu unlösbares technisches Problem und eine unwirtschaftliche Maßnahme.

Das erfindungsgemäße Verfahren zur Herstellung hochkonzentrierter flüssiger Aminoxide beinhaltet die an sich bekannte Reaktion der Oxidation von tertiärem Amin der allgemeinen Formel $R^1R^2R^3N$, worin $R^1$, $R^2$ und $R^3$ die obengenannte

Bedeutung haben, mit Wasserstoffperoxid in handelsüblicher wäßriger 20 bis 90 %iger Lösung. Das Verfahren bedarf nicht der Zugabe eines organischen Lösungsmittels. Es ist jedoch vorteilhaft, ein Sequestriermittel zum Binden von Schwermetallspuren, wie beispielsweise ein Ethylendiamintetraessigsäuresalz, hinzuzusetzen.

Die Herstellung der als Ausgangsprodukte eingesetzten tertiären Amine erfolgt in an sich bekannter Weise durch Alkylierung von sekundären Fettaminen mit Formaldehyd, Acetaldehyd oder Ameisensäure nach der Leuckart-Wallach-Reaktion oder durch reduktive Methylierung mit Formaldehyd oder Acetaldehyd und mit Wasserstoff in Gegenwart von Übergangsmetallkatalysatoren wie Nikkel, Kupfer, Kupferchromit oder Edelmetallkatalysatoren wie Palladium. Die Katalysatoren können dabei vom Raney-Typ sein oder auch auf Träger aufgebracht verwendet werden. Die Einführung einer Hydroxyethyl- oder Hydroxypropyl-Gruppe erfolgt in ebenfalls bekannter Weise durch Umsetzen von sekundären Fettaminen mit Ethylen oder Propylenoxid. Die Herstellung der Polyoxethylen-Gruppen in den Resten $R^1$ und $R^2$ enthaltenden tertiären Amine ist gleichfalls bekannt.

Die als Ausgangsprodukte eingesetzten tertiären Amine der Formel $R^1R^2R^3N$ sind solche, in denen $R^1$ und $R^2$, gleich oder verschieden, Alkyl- oder Alkenylreste mit 8 bis 18 C-Atomen oder Reste der Formel $R'(OCH_2CH)n$ , in der $R'$ für Alkyl- oder Alkenylreste mit 8 - 18 C-Atomen steht 1 bis 5 ist, bedeuten sowie $R^3$ = $CH_3$, $C_2H_5$, $C_2H_4OH$ oder $C_3H_6OH$ ist. Unter Alkenylresten sollen dabei einfach ungesättigte Reste verstanden werden, wobei in bestimmten Gemischen - je nach den vorhandenen Ausgangsfettsäureresten - auch 2 oder 3 Doppelbindungen in untergeordnetem Maße anwesend sein können. Bevorzugt sind diejenigen tertiären Amine, in denen $R^1$ und $R^2$ ein Alkyl- oder Alkenylrest ist, insbesondere auch Gemische solcher Alkyl- oder Alkenylreste, die in der Verteilung nativer Fettsäuren oder Fettsäureschnitte vorliegen. Besonders bevorzugt sind tertiäre Amine, in denen die längeren Alkylreste $R^1$ und $R^2$ eine Kettenlänge von 8 bis 10 C-Atomen aufweisen, wobei $R^1$ und $R^2$ im Gemisch auch mehrere dieser Bedeutungen annehmen können. Besonders bevorzugt sind auch diejenigen tertiären Amine, in denen $R^3$ = $CH_3$ ist.

Als solche speziell geeigneten Ausgangsamine seien hervorgehoben Octyldecyl-, Dioctyl- und Didecylmethylamin, daneben auch die entsprechenden tertiären Amine mit Ethyl-, Hydroxyethyl- oder Hydroxypropylrest als $R^3$. Diese Amine kommen auch vorwiegend im technischen $C_8$-$C_{10}$-Cocosschnitt vor. Ferner sind weitere geeignete Reste - vorzugsweise in Verbindung mit $R^3$ = Methyl - Gemische, wie sie der nativen $C_8$-$C_{18}$-Cocosfettsäure entsprechen, oder auch andere Schnitte daraus, wie zum Beispiel der $C_{12}$-$C_{18}$- oder der $C_{12}$-$C_{14}$-Schnitt, oder auch die $C_{16}$-$C_{18}$-Verteilung, die sich von der Talgfettsäure (mit ungesättigten Anteilen) oder der hydrierten Talgfettsäure ableiten. Ferner können auch Amine, die auf der Basis von kettenreinen Fettsäuren und -alkoholen,wie solche mit Lauryl-, Myristyl-, Cetyl- und Stearylresten, hergestellt wurden, Verwendung finden, ebenso wie solche auf der Basis von chemisch reiner oder technischer Ölsäure (wobei letztere auch Reste mit 2 oder 3 Doppelbindungen enthält). Schließlich sind noch zu nennen tertiäre Amine [$C_8$-$C_{18}$-$(OCH_2CH_2)n$]$_2NR^3$, worin $R^3$ wiederum vorzugsweise $CH_3$ und n = 1 bis 5 ist, und wobei der $C_8$-$C_{18}$-Rest wiederum den nativen Cocos-, Talgfett- oder den hydrierten Talgfett-Rest darstellt. Schließlich können die Reste $R^1$ und $R^2$ auch synthetischen Alkoholen des oben definierten C-Zahlbereichs entstammen,wie sie durch die Ziegler- oder Oxosynthese gewonnen werden, wobei die nach dem letzteren Verfahren hergestellten höhere Anteile an verzweigten Ketten enthalten.

Die Oxidation der tertiären Amine nach dem erfindungsgemäßen Verfahren erfolgt in an sich bekannter Weise mit Wasserstoffperoxid zum entsprechenden Aminoxid. Das Wasser-stoffperoxid wird in Form von handelsüblichen wäßrigen Konzentrationen im Bereich von 20 bis 90 Gew.-% angewendet, die erhaltenen Aminoxide stellen sich dann automatisch je nach Kettenlänge als 40 bis 98 gew.-%ige Lösungen ein.

Bei der Herstellung kann man beispielsweise wie folgt vorgehen: Tertiäres Amin und eine übliche Menge eines Sequestrierungsmittels (beispielsweise Ethylendiamintetraessigsäure, Na-Salz) werden bei Raumtemperatur in eine Rührkolben oder Rührkessel vorgelegt und unter Rühren auf ca. 50 °C erwärmt. Innerhalb 1 bis 3 Stunden wird eine leicht im Überschuß befindliche Menge (102 bis 110 Mol-%) an wäßriger Wasserstoffperoxid-Lösung (20 bis 90 gew.-%ig) so zugetropft, daß die leicht exotherme Reaktion im Temperaturbereich zwischen 60 und 80 °C gehalten werden kann. Gegebenenfalls muß gekühlt werden. Anschließend wird dieses Gemisch noch 8 bis 12 Stunden bei 60 bis 80 °C weitergerührt, bis ein kompletter Umsatz erreicht ist. Überschüssiges Wasserstoffperoxid kann gegebenenfalls auf bekannte Weise zerstört werden, beispielsweise durch Zugabe von NaOH und thermische Zersetzung sowie anschließende Neutralisation mit Mineral- oder organischen Säuren.

Die Reaktion ist ohne Schwierigkeiten beherrschbar und bedarf lediglich der Temperaturkontrolle. In keiner Phase der Reaktion wird eine Gelbildung oder ein gefährlicher Anstieg der Viskosität beobachtet.

Der Gehalt an Aminoxid und Wasserstoffperoxid kann durch gängige Analysenverfahren, wie beispielsweise eine Titration mit TiCl$_3$/NH$_4$Fe(SO$_4$)$_2$ oder mit KI/Thiosulfat bestimmt werden.

Mit steigender Kettenlänge, beispielsweise beim Distearylmethyl-oder Dicocosmethylaminoxid, werden die 60 bis 90 %igen Einstellungen des Aminoxids bei Raumtemperatur als homogene, gießbare, pastöse Flüssigkeiten erhalten. Dioctyl-, Didecyl- und Dioctyldecylmethylaminoxide dagegen sind sowohl bei der Herstellung als auch bei der Aufbewahrung bei Raumtemperatur leicht beweglich, klare Flüssigkeiten.

Die erfindungsgemäß hergestellten Aminoxide sind besonders geeignet als antistatische Zusätze zu Präparationsmitteln für Synthesefasern, wie beispielsweise für Polyester-, Polyamid-, Polyacrylnitril- oder Polyolefinfasern. Solche Präparationsmittel bestehen üblicherweise aus 20 bis 60 Gew.-% eines Gleitmittels, wie beispielsweise Esteröle, Mineralöle, endverschlossene Oxethylate, Propylen-Ethylenoxid-Mischpolymerisate, ferner aus 30 bis 70 Gew.-% eines Emulgators, meist aus der Gruppe der oxethylierten Fettalkohole oder oxethylierten Fettsäuren, gegebenenfalls aus 0,5 bis 5 Gew.-% eines anderen Antistatikums, wie beispielsweise Phosphatestern von C$_4$–C$_{18}$-Fettalkoholen, sowie 1 bis 40, vorzugsweise 5 bis 30 Gew.-% eines Aminoxids, hergestellt nach dem erfindungsgemäßen Verfahren, als Antistatikum.

Die erfindungsgemäß hergestellten Aminoxide haben durch ihren nichtsalzartigen Charakter eine sehr geringe Faden-Metall-Reibung und erzeugen damit im Gegensatz zu den salzartigen Antistatika (wie beispielsweise Alkansulfonate) keine zusätzliche elektrostatische Aufladung durch erhöhte Reibung. Im Gegensatz zu salzartigen Antistatika bilden sie bei der Thermohydrolyse auch keine Salze, die durch Kristallitbildung unerwünschte Schneidvorgänge auf Fadenleitorganen erzeugen können.

Die Erfindung wird durch folgende Beispiele erläutert:

Beispiel 1

651 g (2,5 mol) Dioctylmethylamin (95 Gew.-% Dioctylmethylamin, 4 Gew.-% Octyldecylmethylamin und 0,5 Gew.-% Didecylmethylamin) werden mit 0,1 g Ethylendiamintetraessigsäure, Na-Salz, auf 50 °C erhitzt, und dann werden in 1 h 255,0 g (2,6 mol; 105 Mol-%) 35 gew.-%iges wäßriges Wasserstoffperoxid zugegeben. Durch die exotherme Reaktion steigt die Temperatur auf 60 bis 80 °C. Das Reaktionsgut wird weitere 8 h auf einer Temperatur von 80 °C gehalten. Die Titration zeigt, daß man ein 77,4 gew.-%iges Aminoxid erhält (Ausbeute >99 %), der Restgehalt an Wasserstoffperoxid beträgt 0,4 Gew.-%. Durch Zugabe von NaOH und Nachreaktion bei 60 °C kann dieser Restgehalt unter 0,1 Gew.-% gesenkt werden. Die Dioctylmethylaminoxidlösung ist klar und farblos. Sie besitzt eine Viskosität von 230 mPa s bei 25 °C (Brookfield-Viskosimeter LVT; Drehzahl: 50 min$^{-1}$, Spindel 4).

Beispiel 2

700 g (2,4 mol) Octyldecylmethylamin (22 Gew.-% Dioctylmethylamin, 48 Gew.-% Octyldecylmethylamin und 26 Gew.-% Didecylmethylamin) werden mit 240 g (2,44 mol; 102 Mol-%) Wasserstoffperoxid (35 gew.-%ig) und 0,1 g Ethylendiamintetraessigsäure, Na-Salz, wie in Beispiel 1 erläutert, umgesetzt. Man erhält in 99 %iger Ausbeute ein Octyldecylmethylaminoxid als 79 gew.-%ige wäßrige Lösung mit einer Viskosität von 180 mPa s. Der H$_2$O$_2$-Restgehalt liegt bei 0,3 %.

Beispiel 3

715 g (2,3 mol) Didecylmethylamin (82 Gew.-% Didecylmethylamin, 14 Gew.-% Octyldecylmethylamin und 1 Gew.-% Dioctylmethylamin) werden mit 0,1 g Ethylendiamintetra essigsäure, Na-Salz, versetzt und, wie unter Beispiel 1 beschrieben, mit 235 g (2,5 mol; 105 Mol-%) Wasserstoffperoxid (35 gew.-%ig) 10 h lang bei 80 °C in Reaktion gebracht. Man erhält in 98 %iger Ausbeute ein Didecylmethylaminoxid als 76 gew.-%ige wäßrige Lösung (Viskosität 230 mPa s). Der H$_2$O$_2$-Restgehalt liegt bei <0,1 %.

Beispiel 4

528 g (1,7 mol) Didecylmethylamin werden mit 0,1 g Ethylendiamintetraessigsäure, Na-Salz, auf 50 °C erhitzt, dann werden innerhalb 1 h 86,7 g (1,78 mol; 105 Mol-%) Wasserstoffperoxid (70 gew.-%ig) zugetropft. Die Temperatur steigt auf 80 °C, bei der die Reaktion noch 12 h belassen wird. Man erhält in 98 %igem Umsatz eine Didecylmethylaminoxidlösung als 90 gew.-%ige Lösung in Wasser (Viskosität 240 mPa s). Der H$_2$O$_2$-Restgehalt liegt bei 0,4 %.

Beispiel 5

590 g (1,7 mol) Didecylhydroxyethylamin werden mit 0,1 g Ethylendiamintetraessigsäure, Na-Salz, auf 70 °C erhitzt, dann werden innerhalb 1,5 h 86 g (1,78 mol; 105 Mol-%) Wasserstoffperoxid (70 gew.-%ig) zugetropft. Man rührt 12 h bei 80 °C nach und erhält in 88 %iger Ausbeute ein Didecylhydroxyethylaminoxid als 90 gew.-%ige Lösung in Wasser (Viskosität 720 mPa s). $H_2O_2$-Restgehalt <0,1 %.

### Beispiel 6

525 g (1,5 mol) Didecylhydroxypropylamin werden mit 0,1 g Ethylendiamintetraessigsäure, Na-Salz, auf 70 °C erwärmt und mit 75 g (1,6 mol; 106 Mol-%) Wasserstoffperoxid (70 gew.-%ig) in 1,5 h versetzt und 12 h lang bei 80 °C weitergerührt. Man erhält in 86 %iger Aus-beute ein Didecylhydroxypropylaminoxid als 92 gew.-%ige Lösung in Wasser (Viskosität 430 mPa s). Der $H_2O_2$-Restgehalt liegt bei 0,7 %.

### Beispiel 7

481 g (0,5 mol) Di-$(C_8/_{18})$-cocospentaoxethylmethylamin $[C_8/_{18}H_{17}/_{37}O-(C_2H_4)_5]_2NCH_3$, MG = 962, werden mit 0,1 g Ethylendiamintetraessigsäure, Na-Salz, auf 70 °C erhitzt, dann werden in 1 h 20,8 g (0,55 mol) Wasserstoffperoxid 90 %ig zugetropft und bei 80 °C weitere 10 h gerührt. Die Ausbeute beträgt 97 %. Man erhält ein Dicosospentaoxethylmethylaminoxid als 98 gew.-%ige Lösung (Viskosität 540 mPa s); der $H_2O_2$-Restgehalt liegt bei <0,1 %.

### Beispiel 8

202,5 g (0,5 mol) Di-$(C_{12}/_{18})$cocosmethylamin $(C_{12}/_{18}$-cocosschnitt) und 0,1 g Ethylendiamintetraessigsäure, Na-Salz, werden mit 60 g (0,6 mol) $H_2O_2$ (35 gew.-%ig), wie unter Beispiel 1 angegeben, umgesetzt. Man erhält in 92 %iger Ausbeute eine 74 gew.-%ige Lösung von Dicocosmethylaminoxid (Viskosität 480 mPa s/30 °C), der $H_2O_2$-Restgehalt liegt bei 0,3 %.

### Beispiel 9

134,4 g (0,25 mol) Di-$(C_{16}/_{18})$-stearylmethylamin $(C_{16}/_{18}$-hydrierter Talgfettsäure-Schnitt) und 0,1 g Ethylendiamintetraessigsäure, Na-Salz, werden mit 26,7 g (0,28 mol) $H_2O_2$ (35 gew.-%ig), wie in Beispiel 1 beschrieben, umgesetzt. Man erhält in 94 %iger Ausbeute eine 75 gew.-%ige Lösung von Distearylmethylaminoxid (Viskosität 200 mPa s/ 75 °C); der $H_2O_2$-Restgehalt liegt bei 0,2 %.

## Ansprüche

1. Verfahren zur Herstellung von flüssigen, hochkonzentrierten Aminoxiden durch Oxidation der entsprechenden tertiären Amine mit Wasserstoffperoxid in wäßriger Phase ohne Zusatz organischer Lösungsmittel und ohne Auftreten einer Gelphase, dadurch gekennzeichnet, daß ein tertiäres Amin der Formel $R^1R^2R^3N$, worin $R^1$ und $R^2$, gleich oder verschieden, Alkyl- oder Alkenylreste mit 8 bis 18 C-Atomen oder Reste der Formel $R'(OCH_2CH_2)n'$ in der R' für Alkyl- oder Alkenylreste mit 8 bis 18 C-Atomen und n = 1 bis 5 ist, bedeuten, sowie $R^3$ = $CH_3$, $C_2H_5$, $C_2H_4OH$ oder $C_3H_6OH$ ist, oxidiert wird und die Konzentration des Wasserstoffperoxids so gewählt wird, daß das Aminoxid-Endprodukt der Formel $R^1R^2R^3N\rightarrow O$ in einer Konzentration von 40 bis 98 Gew.-% im Wasser vorliegt.

2. Verfahren zur Herstellung von flüssigen, hochkonzentrierten Aminoxiden nach Anspruch 1, dadurch gekennzeichnet, daß ein tertiäres Amin oxidiert wird, dessen Reste $R^1$ und $R^2$, gleich oder verschieden, Alkylreste mit 8 bis 10 C-Atomen sind, wobei $R^1$ und $R^2$ im Gemisch auch mehrere dieser Bedeutungen annehmen können.

3. Verfahren zur Herstellung von flüssigen, hochkonzentrierten Aminoxiden nach einem oder mehreren der Ansprüche 1 bis 2, dadurch gekennzeichnet, daß ein tertiäres Amin oxidiert wird, in dem $R^3$ = $CH_3$ ist.

## Claims

1. A process for preparing liquid, highly concentrated amine oxides by oxidizing the corresponding tertiary amines with hydrogen peroxide in aqueous phase without addition of organic solvents and without occurrence of a gel phase, characterised by oxidizing a tertiary amine of the formula $R^1R^2R^3N$, where $R^1$ and $R^2$, identical or different, denote alkyl or alkenyl radicals having 8 to 18 carbon atoms or radicals of the formula $R'(OCH_2CH_2)n$, where R' denotes an alkyl or alkenyl radical having 8 to 18 carbon atoms and n is 1 to 5, and $R^3$ is $CH_3$, $C_2H_5$, $C_2H_4OH$ or $C_3H_6OH$, and choosing the concentration of the hydrogen peroxide in such a way that the amine oxide end product of the formula $R^1R^2R^3N\rightarrow O$ is present in the water in a concentration of 40 to 98 % by weight.

2. The process for preparing liquid, highly concentrated amine oxides as claimed in claim 1, characterised in that the tertiary amine oxidized has radicals R¹ and R² which, identical or different, are alkyl radicals having 8 to 10 carbon atoms and, when mixed, can also have more than one of these meanings.

3. The process for preparing liquid, highly concentrated amine oxides as claimed in either of claims 1 and 2, characterised in that the tertiary amine oxidized has an R³ which is CH₃.


**Revendications**

1. Procêdê pour prêparer des amine-N-oxydes liquides très concentrés, par oxydation des amines tertiaires correspondantes au moyen du peroxyde d'hydrogène, en phase aqueuse, sans addition de solvant organique ni formation d'une phase gélifiée, procédé caracterisé en ce qu'on oxyde une amine tertiaire répondant à la formule R¹R²R³N dans laquelle R1 et R2 représentent chacun, indépendamment l'un de l'autre, un radical alkyle ou alcényle contenant de 8 à 18 atomes de carbone ou un radical de formule R' (OCH₂CH₂)n dans lequel R' représente un radical alkyle ou alcényle contenant de 8 à 18 atomes de carbone et n désigne un nombre de 1 à 5 et R³ représente un radical CH₃, C₂H₅, C₂H₄OH ou C₃H₆OH , et on choisit la concentration du peroxyde d'hydrogène de telle façon que l'oxyde d'amine final de formule R¹R²R³N→ O se trouve à une concentration de 40 à 98% en poids dans l'eau.

2. Procédé pour préparer des amine-N-oxydes liquides très concentrés selon la revendication 1, procédé caractérisé en ce qu'on oxyde une amine tertiaire dont les radicaux R¹ et R², qui peuvent être identiques ou différents, sont chacun un alkyle contenant de 8 à 10 atomes de carbone, R¹ et R² pouvant également prendre, dans le mélange, plusieurs de ces significations.

3. Procédé pour préparer des amine-N-oxydes liquides très concentrés selon l'une des revendications 1 et 2, procédé caractérisé en ce qu'on oxyde une amine tertiaire dans laquelle R³ représente CH₃.